# EUROPEAN PATENT APPLICATION

(11) **EP 4 179 970 A1**
(43) Date of publication of application: **17.05.2023**
(21) Application number: 22185211.4
(22) Date of filing: 15.07.2022
(51) Int. Cl.: A61B 5/11, A61H 3/02, A45B 3/08

(54) **SENSOR DEVICE FOR GAIT MONITORING**

(30) Priority: 16.07.2021 ES 202130682
(71) Applicant: Universidad del País Vasco/Euskal Herriko Unibertsitatea, 48940 Leioa, Vizcaya (ES)
(72) Inventor: RODRIGUEZ LARRAD, Ana, 48940 Vizcaya (ES); ZUBIZARRETA PICO, Asier, 48940 Vizcaya (ES); CABANES AXPE, Itzíar, 48940 Vizcaya (ES); BRULL MESANZA, Asier, 48940 Vizcaya (ES); SESAR GIL, Iñigo, 48940 Vizcaya (ES); TORRES UNDA, Juan José, 48940 Vizcaya (ES); PORTILLO PEREZ, EVA, 48940 Vizcaya (ES); ESPÍN, Ander, 48940 Vizcaya (ES); SANTISTEBAN, Leire, 48940 Vizcaya (ES)
(74) Representative: Balder IP Law, S.L.

(57) **Abstract**

The invention relates to a sensor device configured for being coupled to an end of a gait assistive element of a user, the sensor device comprising:
- a casing ,
- at least one force sensor,
- at least one accelerometer and a gyroscope,
- a microcontroller,
the force sensor and the microcontroller being connected to one another and located inside the casing, and configured for monitoring the movement of the user and of the gait assistive element;
- force transmission means for transmitting a force exerted by the user on the gait assistive element to the force sensor;
the sensor device further comprising:
- a battery integrated inside the casing and rechargeable from outside the casing.

## Description

### TECHNICAL FIELD

The present invention is comprised in biomedical engineering, in assistive devices for people who require assistance while walking such as, for example, crutches, walking sticks, walking frames, or the like, and in certain types of prostheses such as lower limb prostheses including, among them, transtibial prostheses. More specifically, the present invention falls within the field of sensor devices for monitoring a user's mobility or gait.

### BACKGROUND OF THE INVENTION

Gait monitoring is a proven area of interest in the health sector given that it allows offering information about physical activity level, is closely correlated with health parameters, and has a bearing on the prediction of relevant variables such as risk of hospitalisation, disability, and even mortality. As will be set forth below, current gait monitoring systems are not satisfactory for people who require assistive devices such as crutches or walking sticks or other complementary assistive devices for walking.

The existing technological solutions for gait monitoring are classified into four major groups: sensorised carpets or mats, 3D motion capture systems, wearable sensors, and sensorised crutches.

Sensorised carpets and mats are fixed devices which are placed on flat surfaces and allow detecting the movement and pressure exerted by the user when he or she walks on said carpets and mats. This monitoring involves two technologies: 1) Belts based on the detection, by means of incorporated pressure/force sensors, of the force exerted upon walking thereon. This technology allows characterising both kinematic parameters of the gait (movement, step pace, etc.) and the weight distribution in each step; and 2) Belts based on photoelectric sensors which only allow the kinematic parameters of the gait to be determined. These products are conceived for the purpose of defining a path across a large space in order to perform tests and they allow the gait to be precisely characterised. However, its usefulness in the continuous monitoring of a person is limited given that it only allows gait monitoring in an enclosed, controlled environment, never outside it.

On the other hand, vision-based 3D motion capture systems are based on the acquisition and the attainment of images through cameras incorporated in the environment for subsequently extracting, and by means of using artificial vision algorithms, the movement of the different limbs of a person's body. The existing devices can be divided into two categories: 1) Those devices which require the incorporation of markers located on a person's body and devices that a person can carry around (such as crutches or walking sticks), such that the cameras detect the relative position of the markers. These systems have a high precision but require a meticulous calibration method. 2) And those devices which do not require markers and use artificial vision algorithms to detect patterns (such as a person's body) and/or to detect movement. Systems of this type have a lower precision compared to the preceding group. In any case, these vision-based systems are limited to the area covered by the cameras which additionally require a direct line of sight with the markers without interferences from objects in the environment. On the other hand, the capturing of images may present privacy problems.

In other words, both sensorised carpets and 3D motion capture systems only allow gait monitoring in a specific space and not outside it. This limits their use for the gait monitoring of the user in a variety of real-life situations, such as climbing up stairs or walking on a slope.

Another common strategy used in monitoring patients' movement is the use of sensors incorporated on the patient's clothing or body. Unlike the two preceding technologies, these wearable sensors have a small size, so they can be incorporated on the patient's clothing or body, allowing different kinds of information to be collected throughout the day and without being limited to a specific environment or space. In the particular case of gait monitoring, inertial measurement units (IMUs), formed by triaxial gyroscopes, accelerometers, and magnetometers which, when combined with sensor fusion algorithms, allow estimating the orientations, positions, and speeds of a body part to which the sensor is attached, are commonly used. However, the highly portable nature of these devices is related to an increased degree of invasiveness among users: they create discomfort among those users wearing them as they are fixed on the clothing. Furthermore, the sensors brushing against the body itself can be a source of interferences which alter the collected measurements. Furthermore, they have not shown an acceptable degree of precision for asymmetrical gait patterns, which are the type of movement common among those people who require technical walking aids.

Another strategy consists of incorporating sensors on a mobility assistive element such as a crutch, a walking stick, or the like, in order to collect data about the user's interaction with said assistive element, such as the force and loads applied, and to thereby monitor the user's 3D motion. This type of device is highly advantageous because it allows monitoring gait without being restricted to a monitoring area. Furthermore, it is minimally invasive because the sensors are not placed on the person's body or clothing. Lastly, it allows characterising the movement pattern of the people who require the use of a gait assistive element. This gait pattern differs from that of the people who do not require an assistive element. An example of this system is described in patent document WO2011039389. Although this system has the advantage of the load cell being optimally placed at the end of the leg of a crutch, it is only capable of measuring the pressure exerted on the crutch, without providing any data about the movement thereof. Integration of the electronic measuring system is not achieved as it requires a cable laid along the inside of the leg of the crutch and a signal amplifier connected to a battery-powered data acquisition card. Similarly, the only element of this device which is placed at the end of the leg is the load cell. The other necessary elements (electronic measuring system and battery) are incorporated in a belt which is connected to the crutch by means of a cable.

Devices belonging in this group typically monitor the load applied on the crutch and the movement thereof. These measured values are generally captured by a device storing said values and sending same to a capture device through Bluetooth or WiFi.

In reference to the components used in movement measuring devices, strain gauges, which are usually adhered to the leg of the crutch for the purpose of measuring the forces exerted thereon, are commonly used. The use of gauges presents the problem of requiring a proper calibration in view of the deformation that the crutch may sustain and of the existence of strayed measurements due to changes in room temperature.

Accelerometers, gyroscopes, and IMUs which combine both are commonly used for monitoring movement. It is not possible to use only accelerometers for measuring movement given that the measurements thereof are greatly altered when support of the gait assistive element occurs. To enable overcoming this problem and to obtain sufficient data so as to be able to measure movement continuously, gyroscopes which, though having the same problem as accelerometers, provide measurements which can vary with temperature and throughout their service life, are used in a complementary manner. Due to these problems, despite the use of IMUs which incorporate both an accelerometer and a gyroscope, the use of algorithms which take into account accelerometer and gyroscope measurement errors is required.

The article "Sensorized tip for monitoring people with multiple sclerosis that require assistive devices for walking"; A. Brull, A. Zubizarreta, I. Cabanes et al; I Sensors; August 2020 describes a device formed by a sensor sub-device which is placed on the tip of the leg of a crutch. The sub-device is formed by a casing containing several elements, among them a rod which transmits the support force to a force sensor, a barometer, and an inertial measurement unit IMU containing an accelerometer, a magnetometer, and a gyroscope. The sub-device further contains a control board with a processor capable of processing signals coming from the sensors and wirelessly communicating with an external apparatus based on a Bluetooth protocol. Although this system presents an improved integration compared to that of other devices of the state of the art, for its operation, this sensor sub-device must be connected to an external battery by means of a cable connected at a first end to the sub-device and having at its opposite end a USB connector whereby it can be connected to a battery.

Despite presenting a significantly improved integration compared to other devices of the state of the art, the patient must carry the external battery and the cable anyway. This entails several drawbacks such as the need for a supplementary housing to carry the battery, the possibility of said battery being able to fall to the ground or of the cable tangling up with the gait assistive element leading, in a worst case scenario, to the tip of the gait assistive element not contacting the ground with the angle desired by the patient, causing said patient to fall. It should be highlighted that although the sensor device described in the publication "*Sensorized tip for monitoring people with multiple sclerosis that require assistive devices for walking"* is capable of measuring the support force of the gait assistive element much more precisely than other devices of the state of the art, it presents measurement inaccuracies when the support forces are of a small magnitude.

There are prior art documents which contemplate the problem of power system integration. This is the case of US-B1-7969315 which describes a device intended for measuring the stress supported by a human body part. US-B1-7969315 describes measuring, by means of pressure sensors, the pressure exerted on the insole of a patient's shoes for the purpose of measuring the stress supported by the patient's foot. The measurement of this pressure is sent to an electronic system contained in a casing by means of a cable. Said casing contains an electronic system powered by an internal power source and is communicated with an external electronic system secured to a gait assistive element. US-B1-7969315 does not describe that this element is rechargeable or that it is designed for being placed at the end of a gait assistive element. This external electronic system is not designed for being placed at the end of a gait assistive element and performs the functions of triggering a warning alarm when the pressure exceeds a certain predetermined level stored in the same element, among others.

Therefore, it would be advantageous to provide a sensor device that overcomes the limitations of the known techniques.

### DESCRIPTION OF THE INVENTION

The invention relates to a sensor device as defined in claim 1. Preferred embodiments of the device are defined in the dependent claims.

To overcome the drawbacks of the state of the art, the present invention proposes a sensor device which is designed and provided to enable the coupling thereof to a gait assistive element for people in a simple manner. Once coupled, the sensor device can attain data about the movement of and the load applied by the user. The captured data is processed by means of algorithms which allow offering healthcare personnel and the actual user with support information concerning parameters relating to the user's ability to walk, which allows personalising a rehabilitation therapy design and/or knowing the evolution of the pathological process; or in the case that the gait assistive element is a prosthesis, the captured data can provide the specialist (for example, traumatologist) with help concerning the correct positioning of the prosthesis.

The sensor device herein described can be adapted to different crutches, walking sticks, walking frames, and in orthopaedic elements for real-time continuous monitoring of 3D motion and the load applied on said devices. Unlike other solutions, the sensor device can be adapted to said devices and it is not necessary for the patient/user to carry any of the parts thereof on his or her body, such that the device is not invasive. Similarly, the sensor device transmits the data to be captured in a simple manner in a mobile device, using consumption standards. The device can integrate a mechanical force pre-tensioning system to improve force measurement. The sensor device, specifically its microcontroller, has an algorithm which allows estimating 3D motion based on the advancement direction of the user in a manner independent of the placement of the sensor device in the crutch or walking stick to be monitored.

The present invention provides a sensor device configured for being coupled to an end of a leg of a gait assistive element. The sensor device comprises:
- a casing,
- at least one force sensor,
- at least one accelerometer and a gyroscope,
- a microcontroller,
   the force sensor and the microcontroller being connected to one another and located inside the casing, and configured for monitoring the movement of the user and of the gait assistive element;
- force transmission means for transmitting a force exerted by the user on the gait assistive element to the force sensor, typically during the use of the gait assistive element as it contacts the ground;
   the sensor device further comprising:
- a battery integrated inside the casing and rechargeable from outside the casing,
preventing the need for the user to carry an external battery, with the corresponding wiring and discomfort that it entails. In other words, in the sensor device herein described, the battery is advantageously integrated inside the casing.

The accelerometer and the gyroscope can be independent elements or can be part of an inertial measurement unit (or IMU). The device may also comprise a magnetometer which can likewise be an independent element or be part of an IMU.

In certain embodiments, the casing of the sensor device comprises an opening. This opening has suitable dimensions to enable introducing a connector from the outside for the purpose of recharging the battery integrated in the casing. It is also possible for charging to be performed by means of electromagnetic induction.

The battery can be configured for powering at least the microcontroller, the IMU (or the accelerometer and gyroscope), and the force sensor.

In certain embodiments, the sensor device further comprises pre-loading means for the force sensor, such that any force applied on the force transmission means can be detected by the force sensor. The sensor device is therefore even capable of measuring small values of force exerted by the user on the gait assistive element. The pre-loading means for the force sensor may comprise a toroidal washer, where the toroidal washer may have a corrugated surface and an aperture for housing the force transmission means.

The sensor device may comprise at a first end coupling means for coupling with the end of the gait assistive element. The sensor device may also comprise at a second end coupling means for coupling with an elastic element, such that the sensor device is protected when it is supported on a surface, and which furthermore performs an anti-sliding function. This protective elastic element can be, for example, a ferrule or a rubber on the leg.

The coupling means of the sensor device may comprise a cylindrical element which can be inserted into an aperture of the end of the gait assistive element, the cylindrical element comprising a transverse groove configured for allowing the compression of the cylindrical element.

Additionally or alternatively, the coupling means for coupling the device to the leg may comprise a tightening mechanism such as a clamp and a wingnut.

The sensor device may further comprise one or more sensors belonging to the following group: accelerometers, gyroscopes, barometric pressure sensors, magnetometers, inertial measurement units, wherein the one or more sensors are housed in the casing.

In certain embodiments, these one or more sensors are connected to a first circuit board, with at least one force sensor, the accelerometer, and the gyroscope being connected to said first circuit board.

In certain embodiments, the sensor device comprises a second circuit board, for adapting the voltages and managing the charge of the battery.

According to a possible arrangement, the first and second circuit boards are arranged parallel to one another inside the casing. Furthermore, according to a preferred embodiment, there is a connection between the second circuit board and the opening of the casing.

In certain embodiments, the casing has therein a support defining first and second cavities inside the casing. In this embodiment, the force sensor can be secured to the support by its lower portion, being housed in the second cavity, and the battery can be secured to the support by its lower portion, being housed in the first cavity.

Said first and second circuit boards can be located in the first cavity above the battery. This configuration is advantageous for integrating the battery and the different elements forming the sensor device in the casing or the encapsulating element.

The force transmission means may comprise a rod which is configured for interacting with the force sensor. This rod can move in a reciprocal manner and is housed in one end of the sensor device. A correct measurement of the force exerted on the end of the gait assistive element resting on the ground to the force sensor is thereby performed. In certain embodiments, the sensor device further comprises a bushing which is located surrounding the rod; this bushing can be a low-friction plastic bushing which improves force transmission between the rod and the force sensor and enables the sensor to also measure small forces from a value practically equal to zero.

The sensor device may further comprise an elastic element arranged at one end thereof, such that the sensor device is protected when it is supported on a surface.

The battery can be a lithium-polymer battery.

The microcontroller of the sensor device can be of the Nrf52IC type, which is advantageous as it has a low consumption.

The sensor device may further comprise a battery-powered circuit for adapting the voltages and charge. This circuit provides current at least to the microcontroller.

The sensor device may further comprise a signal amplification and adaptation circuit which adapts the signal produced by the force sensor.

The present invention also relates to an assembly formed by a gait assistive element and a sensor device as defined above.

The different aspects and embodiments of the invention defined above may be combined together, provided that they are mutually compatible.

Additional advantages and features of the invention will become apparent from the detailed description that follows and will be particularly indicated in the attached claims.

### BRIEF DESCRIPTION OF THE DRAWINGS

As a complement to the description and to help make the features of the invention more readily understandable, according to practical exemplary embodiments thereof, said description is accompanied by a set of drawings constituting an integral part thereof, which by way of illustration and not limitation represent the following:
Figure 1 shows a longitudinal section of the sensor device.
Figure 2 shows a front perspective of the sensor device according to a first embodiment of the invention.
Figure 3 shows a front perspective of the sensor device according to a second embodiment of the invention.
Figure 4 shows a crutch with the sensor device of the second embodiment coupled thereto.
Figure 5 shows a block diagram of some elements of the sensor device.
Figure 6 shows a graph depicting the experimental force measurement attained with the sensor device of the invention. Figure 7 shows a force measurement attained with an existing sensor device.
Figure 8 shows a diagram of the plane of advancement and of how the position of the crutch is referenced with respect to the plane of advancement.

### DESCRIPTION OF EMBODIMENTS OF THE INVENTION

The present description and the embodiments shown in the figures are particularly suitable for a sensor device or assembly which can be adapted to a gait assistive element, such as a walking stick, or a crutch, or a leg prosthesis, for real-time continuous monitoring of 3D motion and the load applied on the walking stick, crutch, or prosthesis (or another gait assistive element). Unlike other existing solutions, the sensor device herein shown is flexible and can be adapted to the walking stick or crutch customised for each person, minimising the invasiveness thereof. As will be explained below, the sensor device has integrated therein a battery, as well as a mechanism for improving the measurement of a force sensor which is integrated therein and monitors the load applied, and a microprocessor which runs an algorithm that allows 3D motion to be estimated based on the advancement direction of the user and in a manner independent of the placement of the device in the crutch or walking stick to be monitored. The sensor device captures this data and transmits it to a mobile device using low-consumption standards.

Figure 1 shows a longitudinal section view of a sensor device 10 according to the invention. Figure 2 illustrates a possible first embodiment of the sensor device which is configured for being placed at the end of a leg 13 of a gait assistive element, such as a crutch 100 (see Figure 4). In another possible embodiment of the sensor device (as will be discussed below in relation to Figures 3 and 4), the sensor device 10 is secured to the crutch by means of a wingnut.

In reference to Figures 1 and 2, the sensor device 10 is made up of a casing 19 manufactured based on a lightweight material or compound, such as aluminium, providing rigidity to the assembly of the sensor device 10. In the embodiments that are shown, the sensor device 10 is integrated in a cylindrical casing 19 or encapsulating element which provides the required rigidity to the assembly and is made up of a first part 40 and a second part 41. Both the first part 40 and the second part 41 have respective first and second circular-shaped protrusions 42, 43 extending in a perpendicular (radial) manner with respect to the axial axis of the casing, with the outer diameter of these first and second protrusions 42, 43 being greater than the outer diameter of their respective first and second parts 40, 41, such that upon attaching the first and second parts 40, 41 by joining the first and second protrusions 42, 43, the attachment surface is larger than what the attachment surface between the parts of the casing would be if there were no such protrusions. This enables the use of attachment element or elements, for example, screws 44, which attach the first and second parts 40, 41 of the casing 19 to one another. Said first and second parts 40, 41, when attached to one another, define an inner cavity 45. This cavity 45 is divided by a support 28 into a first cavity 50 and a second cavity 51; this support is located in coincidence with the first and second protrusions 42, 43 and is firmly attached to the casing.

A plurality of sensors including, among others, a force sensor 27, is present inside the casing 19. In the embodiment that is shown, there is also an accelerometer, a gyroscope, a magnetometer, and/or a barometric pressure sensor 2 (see Figure 5). One or more of these elements, for example, the force sensor 27, the barometric pressure sensor 2, and the accelerometer, or others, can be part of one same inertial measurement unit 3, IMU (see Figure 5).

As illustrated in Figure 2, the casing 19 has an opening 38 through which a connector 39 can be introduced (see Figure 5). A battery 29 arranged inside the first cavity 50 can be recharged (see Figures 1 and 5) by means of this connector 39, which can be a USB connector or another connector.

The length of the sensor device 10 can vary between 10 and 16 cm; its diameter can vary between 4 and 6 cm, measured as the greatest distance between two points of the periphery of the first protrusion 42 or of the second protrusion 43, whichever distance is larger; however, with the exception of tolerance errors, both diameters are usually the same, where one of them is chosen in this case; and its weight varies between 130 and 160 grams.

In a possible embodiment, the first part 40 of the casing 19 of the sensor device has at one of its ends a cylindrical coupling area 16 including a diametrical groove 60 and two half-cylinders 61, 62 on the sides thereof (see Figure 2). This arrangement is advantageous for the coupling of the sensor device 10 at the free end of the leg 13 of the crutch 100, since the two half-cylinders 61, 62 can move slightly towards the diametrical groove 60, such that the coupling area 16 can be more readily introduced into a hollow free end of the leg of the crutch. Once the coupling area 16 is introduced into the hollow free end of the leg of the gait assistive element, the half-cylinders 61, 62 would try to recover their initial position by pressing on the inner wall of the leg of the crutch with their outer portion, thereby ensuring the securing between the free end of the leg of the crutch and the sensor device.

Another possible embodiment of the coupling is illustrated in Figure 3. The end of the leg of the crutch has a rectangular element (not shown in the figures) projecting from said end and sized for fitting into the diametrical groove 60 (Figure 2). As can be seen in Figure 3, the coupling area 16 is surrounded by a clamp to which a wingnut 37 is coupled. Once the rectangular element is introduced into the diametrical groove 60, the wingnut is rotated such that the diameter of the clamp decreases and such that the clamp compresses the half-cylinders onto the rectangular element, ensuring the securing between the end of the leg of the movement assistive element and the sensor device.

In another possible embodiment also illustrated in Figure 3, the wall of the end of the leg of the crutch is introduced between the inner portion of the clamp and the outer surface of the two half-cylinders 61, 62. A wingnut 37 which, when rotated in one direction or another, decreases or increases the perimeter of the clamp, is coupled to said clamp. In the case that the perimeter of the clamp decreases, a compressive force is exerted on the two half-cylinders 61, 62, with the wall of the leg of the crutch compressing the end of the leg on the half-cylinders 61, 62, thereby ensuring the securing between the end of the crutch and the sensor device.

Regardless of the type of coupling used, once the sensor device 10 is secured to the end of the leg of the crutch, a ferrule 15 is provided for the purpose of protecting the sensor device 10 when the crutch 100 is supported on a surface, usually the ground. The ferrule 15 can be an elastic element made of a conventional rubber for the leg.

As indicated, the battery 29 is housed in the first cavity 50 and mechanically connected to the upper portion of the support 28. The battery 29 has a high energy density and a low weight/volume ratio. In the example that is shown, the battery is a lithium-polymer battery. Preferably, the battery has a capacity above 500 mAh in order to ensure that it is suitably durable. On the other side of the support 28, a force sensor 27 is arranged in the second cavity 51 and mechanically connected to the support 28. This specific arrangement of the battery 29, the support 28, and the force sensor 27 is particularly advantageous because, when a rod 18 applies pressure on the force sensor 27, this pressure does not mechanically affect the battery since the support 28, which is firmly connected to the casing 19, absorbs the possible movement that may be transmitted to the battery 29 (as explained in detail below). At the opposite end of the support 28, there is a first circuit board 6 which may contain, according to measurement needs, one, several, or all of the following elements: an accelerometer, a gyroscope, a barometric pressure sensor 2, a processor/microcontroller 7, and the suitable circuitry which enables communication through the board of these devices. The accelerometer and the gyroscope can be comprised in a single inertial measurement unit IMU 3.

Figure 5 shows the block diagram of some elements of the sensor device, and the interaction between said elements. The force sensor 27 is an analogue signal which is amplified and adapted before being captured by the microcontroller 7, for which the microcontroller 7 will have analogue ports. In an alternative embodiment I, the force sensor 27 has an integrated circuitry, particularly of a digital-analogue converter, such that the microcontroller 7 does not have to have analogue ports, but rather receives a digital signal coming directly from the force sensor.

The signals coming from both the barometric pressure sensor 2 and the accelerometer, as well as the gyroscope (accelerometer and gyroscope illustrated in Figure 5 as part of the inertial measurement unit 3 IMU), are transmitted to the microcontroller 7 according to communication protocols known in the state of the art.

The microcontroller 7 is programmed with the desired algorithm. The microcontroller 7 processes the signals from the sensors according to said algorithm for the purpose of estimating the advancement direction 31 of the user (see Figure 8) who uses the crutch 100. Based on this advancement direction 31 and taking into consideration the vertical axis z (parallel to the vector of gravity), it generates a plane of advancement 32 (shown in Figure 8), based on which it is possible to define a first angle θ, which is the angle formed between the vertical axis z and the projection of the gait assistive device, in this case the crutch 100, in a plane perpendicular to the plane of advancement (plane yz); and the angle ψ which is the angle formed between the vertical axis z and the projection of the crutch in the plane of advancement (plane xz). By knowing the position of the plane of advancement and angles θ and ψ, the 3D position of the gait assistive device is determined.

It should be highlighted that this calculation is not performed in an independent manner with respect to the orientation of the sensor device 10 in the crutch, which is advantageous because, in this manner, the sensor device 10 will not require a complex calibration.

To increase the autonomy of the sensor device 10, the chosen microcontroller 7 is a low-consumption processor, i.e., with a consumption below 100 µA at 3 V; the Nordic nRF5832 processor was used in the example that is shown. This microcontroller does not only process signals coming from the different sensors integrated in the sensor device 10 consuming very little energy, but is capable of communicating with an external apparatus (for example, a mobile telephone) in an efficient manner from the energy viewpoint. One example of this type of communication is the so-called Nordic nRF5832 processor which is capable of communicating with a capture device 12 according to BLE "Bluetooth low energy" connectivity.

The barometric pressure sensor 2 located in the first circuit board 6 operates by measuring air pressure and allows measuring the relative height of the sensor device. As indicated above, in the embodiment that is shown, the inertial measurement unit IMU 3 which, in addition to an accelerometer and a gyroscope, comprises a magnetometer, is included.

To enable placing the first circuit board 6 inside the casing in a mechanically reliable manner, a circuit board having a circular shape is preferably chosen.

The first cavity 50 contains, in addition to the first circuit board 6, a second circuit board 9 (see Figure 5) also with a circular shape and placed in a position parallel to the first circuit board 6 (see Figure 1) and intended for the management of the battery. A first function comprised within said management of the battery is the charging of the battery. To enable receiving the intensity required for charging the battery, the second board 9 has at one of its ends the connector 39, preferably a USB connector, which can be mechanically and electrically connected to another external connector which is introduced into the opening 38 of the casing. In the most common case of a USB connector, two of the terminals are used for transmitting the intensity required for charging the battery, whereas the other two terminals can be used for communicating charging parameters such that the battery is charged efficiently.

The first circuit board 6, or sensor board, is powered by means of an electrical connection existing between the second and first circuit boards. This embodiment is advantageous in view of the possibility of connecting both circuit boards to the battery in an independent manner due to the parallel arrangement of the boards 6, 9. It is also technically possible for both the first and second circuit boards to be electrically connected to the battery 29.

The second circuit board 9 includes a preferably linear voltage regulating circuit, such that suitable voltage is fed to the sensors located in the first circuit board 6 independently of other factors, such as the number of times the battery 29 has been recharged.

Although the first circuit board 6 is electrically powered by the second board 9, given that the circuits are located in different boards, there is no possibility of any electrical interaction between the charging management circuits and the control circuits of the first board. This ensures that the signals corresponding to the measurements attained by the sensors and processed in the processor/microcontroller do not undergo distortion due to the charging intensities of the battery.

The parallel arrangement of the first and second circuit boards 6, 9 has the additional advantage of the space of the first cavity 50 being optimally utilised.

At the end opposite the coupling area 16 for coupling with the crutch, the sensor device 10 has an end 21 with a substantially cylindrical shape inside which there is housed a rod 23 which can move in the axial direction with respect to same. If the crutch is supported on a surface, the rod 23 experiences a force which tends to cause a movement towards the force sensor 27. The signal generated by the force sensor 27 depends on the force with which the rod acts thereon.

Friction between the rod 23 and the substantially cylindrical end 21 of the sensor device 10 during the sliding of the rod 23 entails two problems in certain sensor devices of the state of the art.

The first problem consists of the fact that, due to the dynamic friction which acts in a direction opposite the movement of the rod 23, the force exerted by the rod 23 on the force sensor 27 is different from the force transmitted by the support surface to the rod. This causes the signal generated by the force sensor 27 to correspond to a force that is lower than that exerted by the support surface on the rod 23, which entails a reduced precision in the measurement of the force sensor 27.

The second problem is due to the static friction between the rod and the substantially cylindrical end 21 of the sensor device. From a standby position in which the rod is not in contact with the sensor, due to said static friction, the rod 23 is not capable of moving with respect to the substantially cylindrical end 21 below a specific force level, such that it does not exert any pressure whatsoever on the force sensor, although it is indeed subjected to a certain force by the support surface. This makes it impossible to measure the force exerted on the rod, i.e., the force exerted on the sensor device by the support surface when it is below a specific level.

Similarly, from the position in which the rod 23 has exerted a force on the sensor element 27 because it is subjected to a force by the support surface, which force no longer acts in a certain point in time due to static friction, the rod is not completely separated from the force sensor such that it continues to exert a specific force thereon. This causes the force measurement attained by the force sensor to never go below a specific value.

The sensor device of the present invention solves this problem of the state of the art by means of a) introducing a bushing 22 (typically a low-friction bushing) between the rod 23 and the substantially cylindrical end 21 of the sensor device, and b) by means of introducing a mechanical rod pre-tensioning system. Low-friction is understood to mean a coefficient of static friction of between 0.1×10⁻⁶ and 0.50×10⁻⁶.

The bushing 22 reduces the friction, such that as a result of what has been described above, the precision of the measurement attained by the force sensor 27 increases; the sensor device is therefore capable of reaching force measurement values of below 50 N.

The bushing 22 is preferably made up of a plastic material (such as Teflon, for example).

The mechanical pre-tensioning system comprises a first corrugated toroidal washer 26 the lower portion of which is placed on the surface of the second cavity 51 closer to the substantially cylindrical end 21 of the sensor device. In the upper portion of the first washer 26 there is placed a substantially flat second washer 25 also having a toroidal shape.

The rod 23 of the sensor device, as can be seen in Figure 1, is made up of a cylindrical head 24 having a specific diameter and of an elongated body having a smaller diameter than part of said head. The head 24 of the rod 23 contacts the force sensor 27.

As illustrated in Figure 1, the body of the rod 23 goes through the central apertures of the first and second toroidal washers 26, 25, extending into the substantially cylindrical end 21 and along the inner portion of the low-friction bushing 22.

Figure 1 also illustrates how the second washer 25 comes into contact with the head 24 of the rod, transmitting the force exerted by the first washer 26. This causes the rod 23 to exert a specific pressure, pre-tensioning, on the force sensor 27 even if the support surface does not exert any force on said rod.

Once the force sensor 27 has been pre-tensioned, it is calibrated so that the signal generated by said sensor, when it is subjected only to the force corresponding to the pre-tensioning, is that corresponding to force 0. In this manner, regardless of however small the force subjected to the rod by the support surface through the rubber protecting the sensor device is, said force is transmitted to the force sensor which generates a signal corresponding to said force.

Figure 6 shows the precision with which the sensor device 10 of the invention is capable of measuring the force with which the crutch is supported on a surface. To that end, the sensor device is tested by exerting a force which varies over time and is known on the rod. The graph of said force as a function of time is illustrated with boxes in Figure 6. Simultaneously, the force is measured by means of the force sensor and the electronics of the sensor device of the invention. Said time-dependent force (depicted in Newtons on the Y axis as a function of the number of samples - X axis) is shown with crosses in Figure 6.

As can be seen, both graphs are practically the same. It should be indicated that the test corresponding to Figure 6 demonstrates that the sensor device of the invention is capable of measuring values of force exerted by the surface on which it is supported that are practically zero, while at the same time capable of measuring high force values which may reach up to 600 N according to this test. This was not possible in the existing sensors, as shown in Figure 7, in which it was impossible to measure force values below 50 N (in this Figure 7, force, in Newtons on the Y axis, is depicted as a function of time, in seconds on the X axis).

In this text, the words first, second, third, etc. have been used to describe different devices or elements; it should be considered that the devices or elements are not limited by these words given that said words have been used solely for distinguishing one device or element from another. For example, the first device may have been called the second device, and the second device may have been called the first device without departing from the scope of the present disclosure.

In this text, the word "comprises" and its variants (such as "comprising", etc.) should not be understood in an exclusive sense, i.e., they do not exclude the possibility of that which is described including other elements, steps, etc.

Moreover, the invention is not limited to the specific embodiments described herein, but rather encompasses, for example, the variations that a person with average skill in the art could carry out (for example, in terms of the choice of materials, dimensions, components, design, etc.), within the scope of what may be deduced from the claims.

## Claims

1. A sensor device (10) configured for being coupled to an end of a gait assistive element (100) of a user, the sensor device comprising:
- a casing (19),
- at least one force sensor (27),
- at least one accelerometer and a gyroscope (3),
- a microcontroller (7),
the force sensor (27) and the microcontroller (7) being connected to one another and located inside the casing (19), and configured for monitoring the movement of the user and of the gait assistive element (100);
- force transmission means (22, 23) for transmitting a force exerted by the user on the gait assistive element (100) to the force sensor (27);
the sensor device (10) further comprising:
- a battery (29) integrated inside the casing and rechargeable from outside the casing (19).

2. The sensor device (10) according to claim 1, further comprising pre-loading means (25, 26) for the force sensor (27), such that any force applied on the force transmission means (23) can be detected by the force sensor (27).

3. The sensor device (10) according to any of claims 1-2, wherein the casing (19) comprises an opening (38) adapted for receiving a connector (39) for recharging the battery (29).

4. The sensor device (10) according to any of the preceding claims, wherein the battery (29) is configured for powering at least the microcontroller (7) and the at least one force sensor (27).

5. The sensor device (10) according to any of claims 2-4, wherein the pre-loading means for the force sensor (27) comprise a toroidal washer (26).

6. The sensor device (10) according to claim 5, wherein the toroidal washer (26) has a corrugated surface and an aperture for housing the force transmission means (22, 23).

7. The sensor device (10) according to any of the preceding claims, wherein the sensor device (10) comprises at a first end coupling means for coupling with the end (13) of the gait assistive element (100).

8. The sensor device (10) according to claim 7, wherein the coupling means comprise a cylindrical element (16) which can be inserted into an aperture of the end (13), the cylindrical element (16) comprising a transverse groove configured for allowing the compression of the cylindrical element (16).

9. The sensor device (10) according to any of claims 7-8, wherein the coupling means comprise a tightening mechanism (37).

10. The sensor device (10) according to any of the preceding claims, the sensor device comprising (10) one or more sensors selected from: accelerometers, gyroscopes, barometric pressure sensors, magnetometers, inertial measurement units, wherein the one or more sensors are housed inside the casing (19).

11. The sensor device (10) according to claim 10, wherein the one or more sensors are connected to a first circuit board (6), the at least one force sensor (27) being connected to said first circuit board (6).

12. The sensor device (10) according to any of the preceding claims, further comprising a second circuit board (9) for adapting the voltages and managing the charging (9) of the battery (29).

13. The sensor device (10) according to claim 12, wherein the first and second circuit boards (6, 9) are arranged parallel to one another inside the casing (19).

14. The sensor device (10) according to any of claims 12-13 and claim 3, with there being a connection between the second circuit board (9) and the opening (38).

15. The sensor device (10) according to any of the preceding claims, comprising a support (28) inside the casing (19), defining first and second cavities (50, 51).

16. The sensor device (10) according to claim 15, wherein the force sensor (27) is secured to the support (28) in the second cavity (51) and the battery (29) is secured to the support (28) in the first cavity (50).

17. The sensor device according to any of claims 15 and 16 when they depend on one of claims 12-14, wherein the first and the second circuit boards (6, 9) are located in the first cavity (50) above the battery (29).

18. The sensor device according to any of the preceding claims, wherein the force transmission means (22, 23) comprise a rod (23) configured for interacting with the force sensor (27).

19. The sensor device (10) according to claim 18, wherein the rod (23) is configured for moving in a reciprocal manner and is housed in one end of the sensor device (10).

20. The sensor device (10) according to any of claims 18-19, the sensor device comprising (10) a bushing (22) which is located surrounding the rod (23).

21. The sensor device (10) according to any of the preceding claims, further comprising an elastic element (15) arranged at one end of the sensor device (10).

22. The sensor device according to any of the preceding claims, wherein the accelerometer and the gyroscope are integrated in an inertial measurement unit (3).

23. The sensor device (10) according to any of the preceding claims, wherein the battery (29) is a lithium-polymer battery.

24. An assembly formed by a gait assistive element (100) and a sensor device (10) according to any of the preceding claims.
